(19) Europäisches Patentamt
European Patent Office
Office européen des brevets

(11) Veröffentlichungsnummer: **0 141 174**
**B1**

(12) **EUROPÄISCHE PATENTSCHRIFT**

(45) Veröffentlichungstag der Patentschrift: **26.08.87**

(51) Int. Cl.⁴: **C 09 C 1/00**

(21) Anmeldenummer: **84110724.6**

(22) Anmeldetag: **08.09.84**

(54) **Witterungsbeständige Perlglanzpigmente.**

(30) Priorität: **24.09.83 DE 3334598**

(43) Veröffentlichungstag der Anmeldung: **15.05.85 Patentblatt 85/20**

(45) Bekanntmachung des Hinweises auf die Patenterteilung: **26.08.87 Patentblatt 87/35**

(84) Benannte Vertragsstaaten: **BE CH DE FR GB IT LI NL**

(56) Entgegenhaltungen:
**FR - A - 1 270 320**
**GB - A - 1 417 574**

(73) Patentinhaber: **Merck Patent Gesellschaft mit beschränkter Haftung, Frankfurter Strasse 250, D-6100 Darmstadt (DE)**

(72) Erfinder: **Franz, Klaus-Dieter, Dr., Insterburgerstrasse 12, D-6233 Kelkheim (DE)**
Erfinder: **Kieser, Manfred, Dr., Händelstrasse 39, D-6100 Darmstadt 23 (DE)**
Erfinder: **Stahlecker, Otto, Frankfurter Landstrasse 36, D-6100 Darmstadt (DE)**

**EP 0 141 174 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft witterungsbeständige Perlglanzpigmente auf der Basis von mit Metalloxiden beschichteten Glimmerschuppen.

Perlglanzpigmente auf der Basis von mit Metalloxiden beschichteten Glimmerplättchen werden auf vielen technischen Gebieten, wie z.B. in der Kosmetik, als Pigmente für Nagellacke, Lippenstifte, Puder und dergleichen, aber auch zur Pigmentierung von Kunststoffen und Lacken aller Art verwendet. Insbesondere bei der Einarbeitung dieser Pigmente in organische Polymere, z.B. bei der Verwendung in Lacken, Farben und Kunststoffen, muss man jedoch feststellen, dass die Witterungsbeständigkeit der Polymere durch den Gehalt an Perlglanzpigment verschlechtert wird. Offenbar kommt es durch die kombinierte Einwirkung von natürlichem Licht mit seinem hohen UV-Anteil und von Feuchtigkeit unter der katalysierenden Wirkung der auf den Glimmerplättchen aufgebrachten Metalloxide zu einer raschen Zersetzung der Polymermatrix.

Aus der DE-OS2 215 191 ist es bekannt, solche Pigmente mit einer zusätzlichen Beschichtung mit Methacrylatochromchlorid zu überziehen. Dadurch wird in Beschichtungsfilmen oder Kunststoffen eine gute Witterungsbeständigkeit erreicht. Werden jedoch die Pigmente mit wirksamen Mengen dieses Chromkomplexes beschichtet, so erweist sich die starke grüne Eigenfarbe der Zusatzbeschichtung als nachteilig für den Glanz und die Farbqualität des Pigmentes. Darüberhinaus sind für verschiedene Anwendungen, wie z.B. Lebensmittelverpackungen, Beschichtungen auf Chrombasis unerwünscht.

Es bestand daher die Aufgabe, eine chromfreie farbneutrale witterungsbeständige Beschichtung zu finden, die einen möglichst geringen Einfluss auf den Glanz und die Farbwerte des zugrundeliegenden Pigmentes ausübt.

Es wurde nun gefunden, dass diese Aufgabe durch eine Nachbeschichtung der Pigmente mit einem Polysiloxan in Verbindung mit einer Seltenerdmetall-Verbindung gelöst werden kann.

Gegenstand der Erfindung sind daher Perlglanzpigmente mit verbesserter Witterungsbeständigkeit auf der Basis von mit Metalloxiden beschichteten Glimmerschuppen, die dadurch gekennzeichnet sind, dass die Pigmente über der Metalloxidschicht eine Deckschicht besitzen, die ein Polysiloxan, bestehend aus Polysiloxansegmenten des Typs,

$$R^1-O-\underset{R^4}{\overset{R^2}{\underset{|}{\overset{|}{Si}}}}-O-R^3$$

wobei $R^1$ und $R^3$ gleich $-\underset{R^4}{\overset{R^2}{\underset{|}{\overset{|}{Si}}}}-OR^3$, H, Alkyl oder Polyether und $R^2$ und $R^4$ gleich $-\underset{R^4}{\overset{R^2}{\underset{|}{\overset{|}{Si}}}}-OR^3$, Alkyl, Alkylacrylester, Alkylmethacrylester oder Polyether bedeutet, mit einer Viskosität von etwa 20-20.000 $mm^2 \cdot sec^{-1}$ bei 25 °C und einem Molekulargewicht von etwa 500 bis 5000, und eine Seltenerdmetall-Verbindung enthält, wobei das Polysiloxan etwa 0,1 bis 5 Gew.-% und die Seltenerdmetallverbindung etwa 0,5 bis 5 Gew.-% des Gesamtpigments ausmacht.

Gegenstand der Erfindung ist auch ein Verfahren zur Herstellung von Perlglanzpigmenten mit verbesserter Witterungsbeständigkeit auf der Basis von mit Metalloxiden beschichteten Glimmerschuppen, das dadurch gekennzeichnet ist, dass ein mit Metalloxiden beschichtetes Glimmerschuppen-Pigment in wässeriger Suspension bei weitgehend konstantem pH-Wert mit der wässerigen Lösung eines Polysiloxans, bestehend aus Polysiloxansegmenten des Typs,

$$R^1-O-\underset{R^4}{\overset{R^2}{\underset{|}{\overset{|}{Si}}}}-O-R^3$$

wobei $R^1$ und $R^3$ gleich $-\underset{R^4}{\overset{R^2}{\underset{|}{\overset{|}{Si}}}}-OR^3$, H, Alkyl oder Polyether und $R^2$ und $R^4$ gleich $-\underset{R^4}{\overset{R^2}{\underset{|}{\overset{|}{Si}}}}-OR^3$, Alkyl, Alkylacrylester, Alkylmethacrylester oder Polyether bedeutet, mit einer Viskosität von etwa 20-20.000 $mm^2 \cdot sec^{-1}$ bei 25 °C und einem Molekulargewicht von etwa 500 bis 5000, und der wässerigen Lösung eines Seltenerdmetall-Salzes versetzt wird und dass man danach das Pigment abtrennt, wäscht und trocknet, und wobei das Siloxan und die Seltenerdmetallverbindung in solchen Mengen eingesetzt werden, dass sich im fertigen Pigment ein Gehalt von 0,1 bis 5 Gew.-% Siloxan und 0,5 bis 5 Gew.-% Seltenerdmetallverbindung ergibt.

Schliesslich ist Gegenstand der Erfindung auch die Verwendung dieser Pigmente zur Herstellung von organische Polymere enthaltenden witterungsbeständigen Formulierungen wie Lacke, Farben und Kunststoffe.

Als Ausgangsmaterial für die erfindungsgemässen Pigmente können alle üblichen Perlglanzpigmente auf Glimmerbasis verwendet werden. Dies sind Glimmerplättchen mit einem Durchmesser von etwa 1 bis 200 µm und einer Dicke von etwa 0,1 bis 5 µm, die mit einer Beschichtung von farbigen oder farblosen Metalloxiden, insbesondere $TiO_2$, $Fe_2O_3$, $SnO_2$, $Cr_2O_3$, ZnO und anderen Metalloxiden, allein oder in Mischung in einer einheitlichen Schicht oder in aufeinanderfolgenden Schichten versehen sind. Solche Pigmente sind in zahlreichen Patenten und Patentanmeldungen beschrieben, wie z.B. den deutschen Schutzrechten 1 467 468, 1 959 198, 2 009 566, 2 106 613, 2 214 545, 2 244 298, 2 313 331, 2 429 762 und 2 522 572, und können nach den dort beschriebenen Verfahren hergestellt werden. Solche Pigmente bestehen in der Regel zu etwa 20 bis etwa 60 Gew.-% aus Glimmer, wobei die Metalloxide im wesentlichen den Rest ausmachen.

Zur erfindungsgemässen Nachbeschichtung werden diese Basispigmente in Wasser suspendiert, wobei in der Regel etwa 5 bis etwa 20 Gew.-%ige Suspensionen verwendet werden und mit der Seltenerdmetallverbindung und dem Siloxan beschichtet. Dabei kann die Nachbeschichtung sowohl in einem Schritt als auch in mehreren Schritten durchgeführt werden. Die Seltenerdmetall-Verbindung und das Siloxan liegen in getrennten Lösungen vor und können entweder gleichzeitig oder nacheinander der Pigmentsuspension zudosiert werden.

Als Seltenerdmetall wird vorzugsweise Cer verwendet, das insbesondere als Sulfat in 3- oder 4wertiger Form eingesetzt werden kann. Falls die Nachbeschichtung zweistufig erfolgt, wird zunächst die etwa 5 bis 10 Gew.-%ige Cer-Sulfatlösung der Pigmentsuspension zudosiert, wobei durch gleichzeitige Zugabe einer Base wie z.B. einer Alkalilauge oder Ammoniak der pH-Wert der Suspension bei etwa 5 bis 6 gehalten wird. Dabei wird das Cer in hydroxidischer Form auf das Basispigment aufgefällt. Die Menge der zugegebenen Lösung wird so bemessen, dass sich ein Anteil der Seltenerd-Verbindung am Gesamtpigment von etwa 0,5 bis 3 Gew.-% ergibt, berechnet auf das Oxid.

Es kann dann die zweite Beschichtung mit dem Siloxan erfolgen. Als Siloxane werden vor allem Polyorganosiloxane eingesetzt, insbesondere Polyethersiloxane. Dies sind Blockcopolymere, die aus einem linearen oder verzweigten Polysiloxanblock, z.B. einem Polydimethylsiloxan, und einem oder mehreren Polyether- oder anderen polaren Blöcken, wie z.B. Acryl- oder Methacrylderivaten, aufgebaut sind. Durch die Kombination der unpolaren hydrophoben Polydimethylsiloxan-Segmente mit den polaren Acryl-, Methacryl- oder Polyether-, insbesondere Polyäthylenoxid-Einheiten als Seitenketten stehen Copolymere zur Verfügung, deren Eigenschaften sich als besonders vorteilhaft für die Behandlung der erfindungsgemässen Pigmente herausgestellt hat.

Polysiloxane im Sinne dieser Anmeldung sind demgemäss verzweigte Blockcopolymere bestehend aus Polysiloxansegmenten des Typs,

$$\begin{array}{c} R^2 \\ | \\ R^1\text{-O-Si-O-}R^3 \\ | \\ R^4 \end{array}$$

wobei $R^1$ und $R^3$ gleich $\begin{array}{c} R^2 \\ | \\ \text{-Si-O}R^3 \\ | \\ R^4 \end{array}$, H, Alkyl oder Polyether und $R^2$ und $R^4$ gleich $\begin{array}{c} R^2 \\ | \\ \text{-Si-O}R^3 \\ | \\ R^4 \end{array}$, Alkyl, Alkylacrylester, Alkylmethacrylester oder Polyether bedeutet.

Der Gehalt an Alkylacryl- oder Alkylmethacrylgruppen kann etwa 1 - 10 Gew.-% des gesamten Polysiloxans ausmachen. Die Polymere haben eine Viskosität von etwa 20 - 20.000 $mm^2 \cdot sec^{-1}$ bei 25°C, Molekulargewichte von etwa 500 bis 5000 und sind aufgrund des Verhältnisses von verzweigten zu unverzweigten Alkylketten in den Polyethersequenzen von beispielsweise 50% Ethylenoxid zu 50% Propylenoxid wasser- und öllöslich. Durch Erhöhung der Temperatur oder durch Einstellung eines sauren oder stark alkalischen Milieus können sie leicht aus wässriger Lösung durch partielle Hydrolyse ausgefällt werden.

Das Siloxan wird in einer etwa 1 bis etwa 20 Gew.-%igen wässerigen Lösung bzw. Emulsion eingesetzt. Die Menge der zu der Pigmentsuspension zugegeben Lösung wird so bemessen, dass das in der Lösung enthaltene Siloxan etwa 0,5 bis 5 Gew.-% des Pigments ausmacht. Dabei ist zu beachten, dass nicht die gesamte Siloxanmenge auf dem Pigment abgeschieden wird, sondern ein Teil in Lösung verbleibt. Im beschichteten Pigment finden sich daher etwa 0,1 bis etwa 5 Gew.-% Siloxan.

Zusammen mit dem Siloxan können gegebenenfalls weitere Stoffe auf dem Pigment abgeschieden werden. So hat sich der Zusatz einer geringen Menge einer Alkalisilikatlösung zu der Siloxanlösung bzw. -emulsion gut bewährt. In der Regel werden dazu Mengen von etwa 0,05 bis etwa 0,5 g Alkalisilikat pro g Siloxan eingesetzt.

Neben der Lösung, die das Siloxan und gegebenenfalls Silikat enthält, kann eine weitere Lösung von Metallsalzen zugegeben werden, die in hydroxidischer Form in die Beschichtung eingebaut werden. Es haben sich dazu insbesondere Aluminium und Zink bewährt, die einzeln oder gemeinsam in Mengen von etwa 0,5 bis etwa 5 Gew.-%, bezogen auf das Gewicht des Gesamtpigments und bezogen auf die Oxide, eingesetzt werden.

Im Gegensatz zur Fällung der Seltenerdmetall-Verbindung, die bei einem pH-Wert von etwa 5 bis 6 durchgeführt wird, führt man die zweite Stufe der Nachbeschichtung bei einem pH-Wert von etwa 6 bis 8 durch, der durch Zudosierung einer Base entsprechend der Erstbeschichtung konstant gehalten wird.

Anstelle einer zweistufigen Nachbeschichtung kann jedoch auch eine einstufige Nachbeschichtung durchgeführt werden. Dabei werden gleichzeitig Lösungen der Seltenerdmetall-Verbindung, des Siloxans und gegebenenfalls eines Aluminium- und/oder Zinksalzes der Pigmentsuspension bei einem pH von etwa 5 bis 6 zudosiert. Die Temperatur der Pigmentsuspension ist bei allen Nachbeschichtungen an sich nicht kritisch und kann in dem Temperaturbereich zwischen Gefrier- und Siedepunkt der Suspension gewählt werden. In der Regel arbeitet man jedoch bei Temperaturen zwischen Raumtemperatur und etwa 70°C.

Nach Beendigung der Beschichtung wird in der Regel noch einige Minuten, z.B. etwa 10 bis 100 Minuten, nachgerührt und das Pigment danach abgetrennt, gewaschen und einige Stunden bei Temperaturen von etwa 80 bis 140°C getrocknet. Beim Trocknen werden die als Hydroxide gefällten Metallverbindungen entwässert und liegen dann in der Regel als Oxide bzw. Silikate vor. Die erfindungsgemässen Pigmente weisen in der Nachbeschichtung typischerweise folgende Gehalte auf, jeweils berechnet als Oxid und bezogen auf das Gewicht des fertigen Pigments: Cer: 0,5 bis 5 Gew.-%, Silicium: 0 bis 1 Gew.-%, Aluminium: 0 bis 3 Gew.-%, Zink: 0 bis 3 Gew.-%, Siloxan: 0,5 bis 5 Gew.-%. Die Nachbeschichtung insgesamt sollte in einer Grössenordnung von etwa 0,5 bis 10 Gew.-% liegen.

Die erfindungsgemässen Pigmente sind nach dem Trocknen wie die bekannten Pigmente zu verwenden, so z.B. in der Kosmetik, in Kunststoffen, Lacken und Farben. Aufgrund der verbesserten Witterungsbeständigkeit werden aber vor allem solche Anwendungen bevorzugt, bei denen die Pigmente Witterungseinflüssen ausgesetzt sind, wie z.B. in Autolacken.

*Beispiel 1*

Eine Suspension von 1000 g eines nach dem Verfahren des Beispiels 1 der DOS 2 522 572 hergestellten Glimmerpigments mit silbriger Interferenzfarbe in 10 l Wasser wird bei 60°C gleichzeitig mit einer Lösung von 36,5 g Ce $(SO_4)_2 \times 4\,H_2O$ in 500 ml Wasser einer Lösung von 40 g Siloxan Tego 281 (ein Polysiloxanpolyethercopolymer der Firma Goldschmidt AG) und 10 g Natriumsilikat in 500 ml Wasser und einer Lösung von 47,3 g $AlCl_3 \times 6\,H_2O$ in 500 ml Wasser versetzt, wobei der pH-Wert durch Zugabe einer 5%igen Natronlauge bei 5,5 gehalten wird. Danach wird noch 1 Stunde nachgerührt, abfiltriert, mit 12 l Wasser gewaschen, erneut abfiltriert und über Nacht bei 120°C getrocknet. Man erhält ein Pigment mit gutem Glanz und gegenüber dem Basispigment deutlich verbesserter Witterungsstabilität.

*Beispiel 2*

Eine Suspension von 1000 g eines nach dem Verfahren des Beispiels 2 der DOS 2 522 572 hergestellten Glimmerpigments mit blauer Interferenzfarbe in 10 l Wasser wird bei 60°C zunächst mit der Lösung von 25 g Ce $(SO_4)_2 \times 4\,H_2O$ in 500 ml Wasser versetzt, wobei der pH-Wert durch Zugabe von 5%iger Natronlauge bei 5,5 gehalten wird. Nach etwa 20minütigem Nachrühren werden gleichzeitig eine Lösung von 40 g Siloxan Tego 281 und 10 g Natriumsilikat in 500 ml Wasser und eine Lösung von 47 g $AlCl_3 \times 6\,H_2O$ und 17 g $ZnCl_2$ in 500 ml Wasser zugegeben, wobei der pH-Wert durch Zugabe von 5%iger Natronlauge bei 7,0 gehalten wird. Nach 30minütigem Nachrühren wird abfiltriert, mit Wasser gewaschen und über Nacht bei 120°C getrocknet. Man erhält ein Pigment mit gutem Glanz und hervorragender Witterungsbeständigkeit.

*Beispiel 3*

Eine Suspension von 90 g eines nach dem Verfahren des Beispiels 1 der DOS 2 522 572 hergestellten Glimmerpigments mit silberner Interferenzfarbe in 900 ml Wasser wird zunächst mit der Lösung von 2,2 g Ce $(SO_4)_2 \times 4\,H_2O$ in 100 ml Wasser versetzt, wobei der pH-Wert durch gleichzeitige Zugabe von 5%iger Natronlauge bei 5,5 gehalten wird. Nach 15minütigem Nachrühren werden gleichzeitig eine Lösung von 2,7 g Siloxan Tego 281 und 0,8 g Natriumsilikat in 150 ml Wasser und eine Lösung von 3,1 g $ZnCl_2$ in 150 ml Wasser zugegeben, wobei der pH-Wert durch Zugabe 5%iger Natronlauge bei 7,0 gehalten wird. Nach 60minütigem Nachrühren wird abfiltriert, mit Wasser gewaschen und über Nacht bei 120°C getrocknet. Man erhält ein gut glänzendes, weiches Pigment ohne Nebenfällungen und mit sehr guter Witterungsbeständigkeit.

**Patentansprüche**

1. Perlglanzpigment mit verbesserter Witterungsbeständigkeit auf der Basis von mit Metalloxiden beschichteten Glimmerschuppen, dadurch gekennzeichnet, dass die Pigmente über der Metalloxidschicht eine Deckschicht, besitzen, die ein Polysiloxan, bestehend aus Polysiloxansegmenten des Typs,

$$\begin{array}{c} R^2 \\ | \\ R^1\text{-O-Si-O-}R^3 \\ | \\ R^4 \end{array}$$

wobei $R^1$ und $R^3$ gleich $\begin{array}{c} R^2 \\ | \\ \text{-Si-O}R^3 \\ | \\ R^4 \end{array}$, H, Alkyl oder Polyether und $R^2$ und $R^4$ gleich $\begin{array}{c} R^2 \\ | \\ \text{-Si-O}R^3 \\ | \\ R^4 \end{array}$, Alkyl, Alkylacrylester, Alkylmethacrylester oder Polyether bedeutet, mit einer Viskosität von etwa 20-20.000 $mm^2 \cdot sec^{-1}$ bei 25°C und einem Molekulargewicht von etwa 500 bis 4000, und eine Seltenerdmetall-Verbindung enthält, wobei das Polysiloxan etwa 0,1 bis 5 Gew.-% und die Seltenerdmetallverbindung etwa 0,5 bis 5 Gew.-% des Gesamtpigments ausmacht.

2. Perlglanzpigment nach Anspruch 1, dadurch gekennzeichnet, dass als Seltenerdmetall-Verbindung Ceroxid bzw. -hydroxid vorliegt.

3. Perlglanzpigment nach Anspruch 1 oder 2, dadurch gekennzeichnet, dass neben der Seltenerdmetall-Verbindung noch eine Zink- und/oder Aluminium-Verbindung vorliegt.

4. Perlglanzpigment nach einem der Ansprüche 1 bis 3, dadurch gekennzeichnet, dass in der Deckschicht Silikat enthalten ist.

5. Perlglanzpigment nach einem der Ansprüche 1 bis 4, dadurch gekennzeichnet, dass die Deckschicht etwa 0,5 bis etwa 10 Gew.-% des gesamten Pigments ausmacht.

6. Verfahren zur Herstellung von Perlglanzpigmenten mit verbesserter Witterungsbeständigkeit auf der Basis von mit Metalloxiden beschichteten Glimmerschuppen, dadurch gekennzeichnet, dass ein mit Metalloxiden beschichtetes Glimmerschuppenpigment in wässeriger Suspension bei weitgehend konstantem pH-Wert mit der wässerigen Lösung eines Siloxans, bestehend aus Polysiloxansegmenten des Typs,

$$\begin{array}{c} R^2 \\ | \\ R^1\text{-O-Si-O-}R^3 \\ | \\ R^4 \end{array}$$

wobei $R^1$ und $R^3$ gleich $\begin{array}{c} R^2 \\ | \\ \text{-Si-O}R^3 \\ | \\ R^4 \end{array}$, H, Alkyl oder Polyether und $R^2$ und $R^4$ gleich $\begin{array}{c} R^2 \\ | \\ \text{-Si-O}R^3 \\ | \\ R^4 \end{array}$, Alkyl, Alkyl-

acrylester, Alkylmethacrylester oder Polyether bedeutet, mit einer Viskosität von etwa 20-20.000 $mm^2 \cdot sec^{-1}$ bei 25 °C und einem Molekulargewicht von etwa 500 bis 5000, und der wässerigen Lösung eines Seltenerdmetalles versetzt wird und dass man danach das Pigment abtrennt, wäscht und trocknet, und wobei das Siloxan und die Seltenerdmetallverbindung in solchen Mengen eingesetzt werden, dass sich im fertigen Pigment ein Gehalt von 0,1 bis 5 Gew.-% Siloxan und 0,5 bis 5 Gew.-% Seltenerdmetallverbindung ergibt.

7. Verfahren nach Anspruch 6, dadurch gekennzeichnet, dass als Seltenerdmetall-Salzlösung die Lösung eines Cersalzes eingesetzt wird.

8. Verfahren nach Anspruch 6 oder 7, dadurch gekennzeichnet, dass in einer der Lösungen oder getrennt davon noch die Lösung eines Zink- und/oder Aluminiumsalzes zugegeben wird.

9. Verfahren nach einem der Ansprüche 6 bis 8, dadurch gekennzeichnet, dass in einer der Beschichtungslösungen noch ein Alkalisilikat enthalten ist.

10. Verwendung der Perlglanzpigmente nach Anspruch 1 zur Herstellung von organische Polymere enthaltenden witterungsbeständigen Formulierungen wie Lacke, Farben und Kunststoffe.

**Claims**

1. Pearlescent pigment having improved weathering resistance and being based on mica flakes coated with metal oxides, characterised in that on top of the coat of metal oxide the pigments possess a topcoat which contains a polysiloxane consisting of polysiloxane segments of the type

$R^2$
|
$R^1$-O-Si-O-$R^3$
|
$R^4$

$R^2$
|
where $R^1$ and $R^3$ are -Si-$OR^3$, H, alkyl or polyether
|
$R^4$

$R^2$
|
and $R^2$ and $R^4$ are -Si-$OR^3$, alkyl, alkylacrylic ester
|
$R^4$

alkylmethacrylic ester or polyether, having a viscosity at 25°C of about 20-20.000 $mm^2 \cdot sec^{-1}$ and a molecular weight of about 500 to 5000, and a rare earth metal compound, the polysiloxane representing about 0.1 to 5% by weight and the rare earth metal compound representing about 0.5 to 5% bei weight of the total pigment.

2. Pearlescent pigment according to claim 1, characterised in that the rare earth metal compound is cerium oxide or hydroxide.

3. Pearlescent pigment according to claim 1 or 2, characterised in that a zinc and/or aluminium compound is present in addition to the rare earth metal compound.

4. Pearlescent pigment according to any one of claims 1 to 3, characterised in that silicate is present in the topcoat.

5. Pearlescent pigment according to any one of claims 1 to 4, characterised in that the topcoat accounts for about 0.5 to about 10% by weight of the total pigment.

6. Process for preparing pearlescent pigment having improved weathering resistance and being based on mica flakes coated with metal oxides, characterised in that a mica flake pigment cotaed with metal oxides is treated at substantially constant pH in aqueous suspension with an aqueous solution of a siloxane consisting of polysiloxane segments of the type

$R^2$
|
$R^1$-O-Si-O-$R^3$
|
$R^4$

$R^2$
|
where $R^1$ and $R^3$ are -Si-$OR^3$, H, alkyl or polyether
|
$R^4$

$R^2$
|
and $R^2$ and $R^4$ are -Si-$OR^3$, alkyl, alkylacrylic ester
|
$R^4$

alkylmethacrylic ester or polyether, having a viscosity at 25°C of about 20-20.000 $mm^2 \cdot sec^{-1}$ and a molecular weight of about 500 to 5000, and an aqueous solution of a rare earth metal salt and in that, thereafter, the pigment is separated off, washed and dried, whereby the siloxane and the rare metal compound are used in such amounts that a content of 0.1 to 5% by weight of siloxane and 0.5 to 5% by weight of rare earth metal compound results in the finished pigment.

7. Process according to claim 6, characterised in that the rare earth metal salt solution is a solution of a cerium salt.

8. Process according to claim 6 or 7, characterised in that a solution of a zinc and/or aluminium salt is added in one of the solutions or separate therefrom.

9. Process according to any one of claims 6 to 8, characterised in that an alkali metal silicate is additionally present in one of the coating solutions.

10. The use of the pearlescent pigments according to claim 1 for preparing weathering-resistant formulations containing organic polymers, such as paints, inks and plastics.

**Revendications**

1. Pigment d'un lustre nacré ayant une meilleure stabilité aux agents atmosphériques et à base de paillettes de mica enduites d'oxydes métalliques, caractérisé en ce que les pigments comportent, pardessus la couche d'oxyde métallique, une couche de recouvrement qui contient un polysiloxane constitué de segments de polysiloxane de type

```
     R2
     |
R1-O-Si-O-R3
     |
     R4
```

```
                R2
                |
où R1 et R3 représentent -Si-OR3, H, un groupe alkyle
                |            R2
                R4           |
ou un polyéther et R2 et R4 représentent -Si-OR3, un
                             |
                             R4
```

groupe alkyle, un groupe d'ester alkylacrylique, un groupe d'ester alkylméthacrylique ou un polyéther, ce polysiloxane ayant une viscosité d'environ 20-20.000 $mm^2 \cdot sec^{-1}$ à 25°C et un poids moléculaire d'environ 500 à 5.000, ainsi qu'un composé d'un métal de terre rare, le polysiloxane représentant environ 0,1 à 5% en poids et le composé de métal de terre rare, environ 0,5 à 5% en poids de tout le pigment.

2. Pigment d'un lustre nacré selon la revendication 1, caractérisé en ce que, comme composé d'un métal de terre rare, on a l'oxyde ou l'hydroxyde de cérium.

3. Pigment d'un lustre nacré selon la revendication 1 ou 2, caractérisé en ce que, outre le composé d'un métal de terre rare, on a encore un composé de zinc et/ou d'aluminium.

4. Pigment d'un lustre nacré selon une des revendications 1 à 3, caractérisé en ce que la couche de recouvrement contient un silicate.

5. Pigment d'un lustre nacré selon une des revendications 1 à 4, caractérisé en ce que la couche de recouvrement représente environ 0,5 à environ 10% en poids de tout le pigment.

6. Procédé de préparation de pigment d'un lustre nacré ayant une meilleure stabilité aux agents atmosphériques et à base de paillettes de mica enduites d'oxydes métalliques, caractérisé en ce que, à un pigment en paillettes de mica enduit d'oxydes métalliques, en suspension aqueuse et à un pH essentiellement constant, on ajoute la solution aqueuse d'un siloxane constitué de segments de polysiloxane de type

```
     R2
     |
R1-O-Si-O-R3
     |
     R4
```

```
                R2
                |
où R1 et R3 représentent -Si-OR3, H, un groupe alkyle
                |            R2
                R4           |
ou un polyéther et R2 et R4 représentent -Si-OR3, un
                             |
                             R4
```

groupe alkyle, un groupe d'ester alkylacrylique, un groupe d'ester alkylméthacrylique ou un polyéther, ce polysiloxane ayant une viscosité d'environ 20-20.000 $mm^2 \cdot sec^{-1}$ à 25°C et un poids moléculaire d'environ 500 à 5.000, ainsi que la solution aqueuse d'un métal de terre rare, après quoi on sépare le pigment, on le lave et on le sèche, le siloxane et le composé du métal de terre rare étant utilisés en quantités calculées de telle sorte qu'il y ait, dans le pigment définitif, une teneur de 0,1 à 5% en poids de siloxane et de 0,5 à 5% en poids du composé du métal de terre rare.

7. Procédé selon la revendication 6, caractérisé en ce que, comme solution d'un sel d'un métal de terre rare, on utilise la solution d'un sel de cérium.

8. Procédé selon la revendication 6 ou 7, caractérisé en ce que, dans une des solutions ou séparément de celles-ci, on ajoute encore la solution d'un sel de zinc et/ou d'un sel d'aluminium.

9. Procédé selon une des revendications 6 à 8, caractérisé en ce qu'une des solutions d'enduction contient encore un silicate alcalin.

10. Utilisation des pigments d'un lustre nacré selon la revendication 1 pour la préparation de formulations contenant des polymères organiques et stables aux agents atmosphériques telles que des vernis, des peintures et des matières synthétiques.